(19) Europäisches Patentamt
European Patent Office
Office européen des brevets

(11) Publication number: **0 102 808**
**B1**

(12) **EUROPEAN PATENT SPECIFICATION**

(45) Date of publication of patent specification: **04.02.87**

(51) Int. Cl.[4]: **C 07 C 109/16** // B01J27/32

(21) Application number: **83304887.9**

(22) Date of filing: **24.08.83**

# (54) Process for producing benzophenone-azines.

(30) Priority: **25.08.82 JP 147151/82**

(43) Date of publication of application:
**14.03.84 Bulletin 84/11**

(45) Publication of the grant of the patent:
**04.02.87 Bulletin 87/06**

(84) Designated Contracting States:
**DE FR GB**

(56) References cited:
**EP-A-0 038 708**
**FR-A-2 456 086**

**CHEMICAL ABSTRACTS, vol. 93, no. 15, 13th October 1980, page 693, no. 149996g, Columbus, Ohio, USA**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Nawata, Takanari**
**No. 5-19-3, Kanamachi Katsushika-ku**
**Tokyo (JP)**
Inventor: **Sakaguchi, Shuzabu**
**No. 19-5, Toride-nishi 1-chome**
**Ibaraki (JP)**
Inventor: **Kohzaki, Toshiaki**
**No. 2689-20, Kashiwada Ushikumachi**
**Inashiki-gun Ibaraki (JP)**
Inventor: **Aoki, Osamu**
**No. 69, Takayanagishinden**
**Matsudo-shi Chiba (JP)**
Inventor: **Takeda, Norio**
**277-28, Oaza Obusuma Showamachi**
**Kita-katsushika-gun Saitama (JP)**
Inventor: **Shimpo, Masahumi**
**No. 5-11-16, Kanamachi Katsushika-ku**
**Tokyo (JP)**

(74) Representative: **Diamond, Bryan Clive et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 808 B1

**Description**

This invention relates to a process for producing benzophenone-azines by oxidation of benzophenone-imines with molecular oxygen and, more particularly, to removal, recovery and recycling of a catalyst used therefor.

Benzophenone-azines are important intermediates for the production of hydrazine as disclosed in U.S. Patent 4,239,741, and development of an economical process for the production thereof has been long desired.

Of conventional processes for producing benzophenone-azines comprising contacting benzophenone-imines with molecular oxygen in the presence of a cuprous halide, a process using cuprous chloride as a catalyst is widely known as disclosed in U.S. Patent 2,870,206. In this process, it is desired that the catalyst used be recovered and reused in the production on an industrial scale.

The above-described U.S. Patent 2,870,206 discloses a process wherein aqueous ammonia or an ammonium chloride aqueous solution is brought into contact with the reaction system to extract the catalyst, which is then removed from the reaction system. This U.S. patent, however, does not refer to reuse of the catalyst that would bring about an industrial advantage.

Further, Japanese Patent Applications OPI (Open to Public Inspection) Nos. 71045/78 and 38346/80 disclose processes in which water, aqueous ammonia or a salt solution is added to the reaction system to precipitate a copper salt, which is then recovered. However, these processes involving precipitation of a copper salt are disadvantageous in that troublesome operations inevitably associated with handling of solid, such as filtration, separation of a precipitate or transportation of a slurry, are required.

According to the present invention we provide a process of producing a benzophenone-azine wherein:

(a) a benzophenone-imine is oxidized with molecular oxygen in the presence of at least one copper halide catalyst to produce a benzophenone-azine, and

(b) the oxidation reaction mixture obtained in the step (a) is brought into contact with an inorganic aqueous solution to extract and remove the catalyst;

characterized in that in step (b) the aqueous extractant solution contains (i) a mineral acid at a concentration of 0.1 to 10% by weight and in an amount of 0.5 to 5.0 equivalents based on the benzophenoneimine fed and (ii) an inorganic halide at a concentration of 0.1 to 50% by weight and that

(c) the catalyst-containing aqueous solution obtained in the step (b) is brought into contact with benzophenone-imine, which may be at least partly obtained from that fed to step (a), to extract the catalyst, and the catalyst-containing benzophenone-imine solution obtained is recycled to the step (a) in the form of a benzophenone-imine solution.

The molar ratio of the benzophenone-imines to the copper halide catalyst present in the reaction mixture has a significant relation to resistance of the catalyst against moisture including water fomed as a by-product, i.e., so-called moisture-resistance. More specifically, the present inventors found that, when the above described molar ratio is maintained at 1.5 or more, preferably 2 or more, a continuous reaction can be performed stably and economically for a long period of time even under pressurized conditions without increasing the amount of an oxygen-containing gas which is introduced for oxidation of the benzophenone-imines and at the same time for elimination of water produced.

Accordingly, in one preferred embodiment of this invention, the oxidation of benzophenone-imines with oxygen or an oxygen-containing gas in the presence of a copper halide catalyst is carried out while controlling the conversion of benzophenone-imines so taht the molar ratio of benzophenone-imines to copper halide is 1.5 or more.

Further, in another preferred embodiment of this invention, the unreacted benzophenone-imines remaining in the oxidation reaction mixture obtained in the above step (a) is reacted with water in the presence of a specific oxide or carboxylic acid to hydrolyze the benzophenone-imines into ammonia and benzophenones and, subsequently, the copper halide catalyst is extracted according to the step (b).

The present invention makes it possible to recover and recycle the copper halide catalyst and, consequently, to produce benzophenone-azines with industrial advantages.

Benzophenone-imines that can be used in the present invention are represented by the following formula (I):

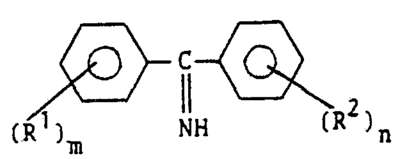

(I)

wherein $R^1$ and $R^2$, which may be the same or different from each other, each represents an acyclic, alicyclic or aromatic hydrocarbon group having not more than 10 carbon atoms, an ether or acyl group derived from these hydrocarbon groups, a halogen atom, a hydroxyl group, a nitro group or a cyano group, or $R^1$ and $R^2$ may be taken together to form a single bond or a ring; and *m* and *n* each represents 0 or an integer of 1 to 5.

Specific examples of the benzophenone-imines represented by the above formula (I) include benzophenone-imine, 2-, 3- or 4-methylbenzophenone-imine, 2-, 3- or 4-ethylbenzophenone-imine, 2-, 3- or 4-*n*- or *iso*-propylbenzophenone-imine, 2-, 3- or 4-*n*- or *iso*- or *tert*-butylbenzophenone-imine, 2-, 3- or 4-aminobenzophenone-imine, 2-, 3- or 4-decylbenzophenone-imine, 2-, 3-, or 4-methoxybenzophenone-imine, 4-phenylbenzophenone-imine, 2,4-dimethylbenzophenone-imine, 2,3-dimethylbenzophenone-imine, 3,4-dimethylbenzophenone-imine, 2,4-diethylbenzophenone-imine, 2,3-diethylbenzophenone-imine, 3,4-diethylbenzophenone-imine, 2-methyl-4-ethylbenzophenone-imine, 2-methyl-4-butylbenzophenone-imine, 2,2'-, 3,3'-, 4,4'-, 2,3'-, 2,4'- or 3,4'-dimethylbenzophenone-imine, 2-, 3- or 4-cholorbenzophenone-imine, 2-chloro-4-methylbenzophenone-imine, 4-chloro-4'-methylbenzophenone-imine, 4,4'-dichloro-benzophenone-imine, 4-nitrobenzophenone-imine, 2,4-dinitrobenzophenone-imine, 4-N,N-dimethylaminobenzophenone-imine, 4-methoxycarbonylbenzophenone-imine, 4-acetylbenzophenone-imine, 4-hydroxybenzophenone-imine, 4-cyanobenzophenone-imine, 4-N,N-dimethylcarbamoylbenzophenone-imine, xanthone-imine, anthrone-imine, acridone-imine or fluorenone-imine.

Of course other benzophenone-imine compounds may also be be used.

Methods for preparing these and other benzophenone-imines include, for example, a method of reacting a corresponding benzophenone with ammonia as disclosed in U.S. Patent 4,083,869, a method of reacting benzonitriles with an aryl-magnesium bromide, i.e., a Grignard reagent, a method of dehydrating diarylamino alcohols, and the like. All benzophenone-imines prepared by any of these methods can be used in the present invention.

The benzophenone-imines that can be used in the present invention except for unsubstituted benzophenone-imine of the formula (I) wherein *m* and *n* are 0 are those containing various substituents or with substituents jointly forming a single bond or a ring. In carrying out the process of the invention on an industrial scale, unsubstituted benzophenone-imine and 1- or 2-mono- or 1,2-di-substituted benzophenone-imines are preferred. Among them, unsubstituted benzophenone-imine is particularly preferred.

The copper halide catalyst that can be used in the present invention preferably includes cuprous chloride, cuprous bromide and cuprous iodide. However, various other copper salts can be employed as long as they are used at the beginning of the production of benzophenone-azines, i.e., in the initial stage of catalyst feeding in the step (a), as well as for making-up for catalyst loss. Such copper salts include cupric chloride, copper oxychloride, copper methoxychloride, cupric bromide, copper oxybromide, copper methoxybromide, cupric iodide, copper oxyiodide and copper methoxyiodide.

Solvents are not particularly required in the present invention. It is possible, however, to use solvents for the purpose of maintaining the reaction system in a solution state or facilitating oil-water separation in the extraction or back extraction. Such being the case, solvents that are not easily oxidized in the oxidation of benzophenone-imines and, in particular, have poor affinity for water and a low viscosity are preferred. Examples of such solvents are aromatic hydrocarbons having 6 to 16 carbon atoms, such as benzene, toluene, *o*-, *m*- or *p*-xylene, ethylbenzene, mesitylene, cumene, pseudocumene, amylbenzene, etc., and mixtures of these aromatic hydrocarbons, chlorobenzene, *o*-, *m*- or *p*-dichlorobenzene, nitrobenzene, *o*-, *m*- or *p*-dinitrobenzene, *o*-, *m*- or *p*-chlorotoluene, diphenyl, phenanthrene, anisole, diphenyl ether, acetophenone, dibenzyl, benzophenone, hexane, heptane, cyclohexane, cyclooctane, ethylcyclohexane, ethylene dichloride, tetrachloroethylene, diisopropyl ether, dipropyl ether, diisobutyl ketone, butyl acetate, butyl benzoate, phenyl benzoate and dimethyl phthalate. Usually, it is preferable to use an imination reaction mixture of benzophenones as the benzophenone-imines, and reaction mixtures having benzophenone-imines concentrations of 1 to 90% by weight, preferably 5 to 50% by weight, are usually used. In case of using the imination reaction mixture, a solvent is not always necessary since the unreacted benzophenones remaining in the reaction mixture serves as a solvent.

Each step of the process of the present invention will now be illustrated in detail.

Step (a):

Conditions for the oxidation of benzophenone-imines are not definitely specified as they vary depending on the active amount of the copper halide catalyst added to the reaction system and the like, but usually, the copper halide catalyst is used in an amount ranging from $10^{-4}$ mol to 2 mols, preferably $10^{-3}$ to 1 mol, per liter of the total volume of the reaction mixture. The reaction temperature usually ranges from 60° to 250°C, preferably from 70° to 230°C. As molecular oxygen, air, pure oxygen and other oxygen-containing gases can be used either under atmospheric pressure or under pressure. The oxygen partial pressure is preferably from 0.01 to 20 atm., more preferably from 0.05 to 10 atm., but wider ranges may also be used. The reaction may be carried out in either a batch system or a continuous system. It is desirable to control the oxidation reaction so that the conversion of imines is 99% or less taking into account side reactions and precipitation of the catalyst.

Step (b):

In the step (b), the oxidation reaction mixture obtained in the step (a) is brought into contact with an aqueous solution containing a mineral acid and an inorganic halide to extract and remove the catalyst from the oxidation mixture.

The mineral acids herein used include, for example, hydrochloric acid, hydrobromic acid, hydroiodic acid, sulfuric acid, phosphoric acid, carbonic acid and nitric acid with hydrochloric acid, hydrobromic acid

and sulfuric acid being preferred. The inorganic halides include, for example, ammonium chloride, ammonium bromide, ammonium iodide, and chlorides, bromides, iodides, etc. of lithium, sodium, potassium, beryllium, magnesium, calcium, and aluminum with ammonium chloride, ammonium bromide, sodium chloride and potassium chloride being preferred.

Concentrations of the inorganic halides in the aqueous solution to be supplied to the extraction system are 0.1 to 50% by weight, preferably 1 to 30% by weight. The water/oil ratio to be used in the extraction operation is not also specified, but preferably ranges from 0.05 to 20 by weight.

By use of an aqueous solution containing a mineral acid and an inorganic halide, the benzophenone-imines can be rapidly hydrolyzed and the copper halide catalyst can be extracted and removed at a good efficiency. The amount of the mineral acid to be used here ranges from 0.5 to 5.0 equivalents, based on the benzophenone-imines fed to the extraction zone.

The extraction temperature, pressure and contact time between oil and water in the step (b) are not particularly restricted, but an illustrative example is 30 to 200°C for temperature, 0.1 to 10 atm. pressure and 1 second to several hours in contact time.

The extraction and removal of the copper halide catalyst in the step (b) may be carried out either in the presence or in the absence of air. When the extraction is performed in the presence of air, the copper halide catalyst may be satisfactorily extracted and removed. In this operation, however, the copper halide catalyst is extracted in the aqueous layer in the form of a cupric ion, which must be reduced to a cuprous ion prior to the extraction of the step (c) since the extraction efficiency of the cupric ion in the step (c) is low. Therefore, it is preferable that the extraction operation in the step (c) is performed in the absence of air. Means for maintaining an air-free extraction system include displacement of the system with gases, such as nitrogen, helium, argon, hydrogen, methane, ethane, carbon dioxide or steam, and sealing the reaction system.

In the step (b) in which the oxidation reaction mixture obtained in the step (a) is contacted with a specific aqueous solution to extract and remove the copper halide catalyst, it is preferably that when the oxidation reaction mixture contains any unreacted benzophenone-imines, a part or the whole of the unreacted benzophenone-imines is hydrolyzed into benzophenones and ammonia, which are then recovered, and thereafter the oxidation solution is brought into contact with the above-described aqueous solution. The hydrolysis of the benzophenone-imines is generally conducted by feeding water or steam to the oxidation reaction mixture and heating the system in the presence of at least one metal oxide catalyst or a carboxylic acid catalyst.

The metal oxide catalyst includes oxides of at least one element selected from the group consisting of elements belonging to the Groups III to V and the Periods 2 to 5 of the Periodic Table (excluding phosphorus, nitrogen and carbon), iron, tungsten, bismuth, cerium and thorium.

These oxides may be used alone or in combination of two or more of them. The oxides do not necessarily require high purity. Therefore, incorporation of other components incapable of decomposing the benzophenone-imines is accepted as long as they do not interfere with the activity of the above-described hydrolyzing catalysts.

The above-described metal oxides may be either naturally-occurring or synthetic and specifically include single-component substances, e.g., boron trioxide, aluminium oxide, silicon oxide, scandium oxide, titanium oxide, vanadium pentoxide, gallium oxide, germanium oxide, diarsenic pentoxide, yttrium oxide, zirconium oxide, niobium oxide, indium oxide, stannic oxide, antimony pentoxide, ferric oxide, tungsten oxide, bismuth oxide, cerium oxide, thorium oxide; two-component substances, e.g. silica alumina, silica zirconia, silica magnesia, silica boria; natural clay substances, e.g., acid clay, bentonite, kaoline and montmorilonite; and synthetic zeolite.

The catalytic activity of the oxides can effectively be improved by, for example, subjecting naturally occurring acid clay to acid-treatment to further increase its activity or converting a usual zeolite to an acid type.

Among these metal oxides, aluminium oxide, silicon oxide, vanadium pentoxide, zirconium oxide, stannic oxide, antimony pentoxide, ferric oxide, tungsten oxide, thorium oxide, silica alumina, silica zirconia, zeolite and activated clay are preferred, with aluminum oxide, silicon oxide, stannic oxide, antimony pentoxide, ferric oxide, silica alumina, silica zirconia, zeolite and activated clay being particularly preferred.

The shape of these oxides is no particularly limited, but particles of several microns or more are preferred in view of ease of separation from the reaction mixture. Powderous oxides are suitably used as dispersed in the reaction mixture, and granular oxides are suitably used in a fixed bed system.

The amount of the metal oxide to be used is not particularly limited. Generally, the oxide is used in an amount of from 0.01 to 100 parts by weight, preferably from 0.1 to 50 parts by weight, per 100 parts by weight of the reaction mixture.

The carboxylic acids that can be used as catalysts in the hydrolysis of the benzophenone-imines with water include aliphatic, alicyclic, aromatic and other carboxylic acids. Illustrated examples of these carboxylic acids are acetic acid, propionic acid, butyric acid, isobutyric acid, valeric acid, pivalic acid, caproic acid, enanthic acid, caprylic acid, pelargonic acid, capric acid, undecanoic acid, lauric acid, tridecanoic acid, myristic acid, palmitic acid, stearic acid, chloroacetic acid, bromoacetic acid, fluoroacetic acid, trichloroacetic acid, trifluoroacetic acid, glycolic acid, lactic acid, oxalic acid, malonic acid, succinic acid, adipic acid, maleic acid, malic acid, citric acid, azelaic acid, phthalic acid, isophthalic acid, terephthalic

acid, trimellitic acid, trimesic acid, nicotic acid, isonicotic acid, benzoic acid, tolilic acid, chlorobenzoic acid, nitrobenzoic acid, salicyclic acid, anthranilic acid, glycine, alanine, methionine and leucine. Compounds which are not in the form of a carboxylic acid but capable of forming carboxylic acids under the reaction conditions may also be included. For example, compounds capable of producing carboxylic acids upon contact with water or upon thermal decomposition can be used. Examples of such compounds are ammonium carboxylate, acid amide, nitrile, esters, acid chlorides and the like. Those compounds that are thermally decomposed at the reaction temperature to produce carboxylic acids may also be used.

The amount of the carboxylic acid to be used in the hydrolysis is not particularly restricted but, in general, ranges from 0.005 to 10% by weight, preferably 0.02 to 2% by weight, based on the oxidation reaction mixture.

The reaction conditions that are employed in the present invention vary depending on the activity of the imine-hydrolyzing catalyst, reaction mode and the like. Generally, the reaction temperature is from 60° to 350°C, preferably 80° to 300°C, more preferably 100° to 270°C, and the reaction pressure is preferably atmospheric pressure to 30 atmospheres. Water is fed in an equimolar or more amount based on the benzophenone-imines.

Step (c):

In this step, the copper halide catalyst contained in the aqueous solution obtained in the step (b) is removed from the aqueous solution and, at the same time, changed so as to be reused in the oxidation reaction of benzophenone-imines in the step (a) and returned to the step (a).

When the copper halide-containing aqueous solution obtained in the step (b) is contacted with the benzophenone-imines, the starting material in the step (a), the copper halide is easily extracted in the benzophenone-imines and the extract can be recycled as a copper halide catalyst for the oxidation of benzophenone-imine in the step (a).

The conditions for this copper halide catlayst extraction vary depending on the concentrations of the copper halide catalyst in the aqueous solution to be extracted, the type of the benzophenone-imines, the presence or absence of organic solvents, the type of the aqueous solution, the method of extraction, and the like and cannot, therefore, be definitely specified. Usually, the extraction temperature ranges from 0° to 200°C, preferably 30° to 150°C, and the extraction time is in the range of from 1 second to 1 hour. The operational atmosphere is not particularly restricted, but a cuprous ion is extracted at a higher efficiency than a cupric ion as described above. Therefore, the extraction is preferably carried out in the absence of air as described with respect to the extraction in the step (b).

The presence of a cupric ion in the aqueous solution to be subjected to extraction causes no inconvenience, but for the purpose of improving extraction efficiency, it is preferable to reduce the cupric ion to a cuprous ion by any means such as reacting with a reducing agent. The reducing agent which can be used for this purpose includes hydrazine, hydrazine salts, hydroxylamine salts, metal copper, sulfurous acid, sulfites, dithionous acids, phosphine, hypophosphites, etc. It is, of course, possible to perform the reduction operation simultaneously with the extraction operation.

The pH value of the aqueous solution to be subjected to extraction is not particularly limited, but when an acid is copresent in the aqueous solution, the co-existing acid may be previously neutralized with an alkali such as ammonia, sodium hydroxide, potassium hydroxide, lime and sodium carbonate, in order to suppress the formation of benzophenone-imine salts or hydrolysis of benzophenone-imines.

The amount of the benzophenone-imines to be used as an extracting solvent in this step may be the whole or a part of the benzophenone-imines which is subject to the oxidation reaction for obtaining a benzophenone-azines. In the latter case, it is also possible that the concentration of the copper halide catalyst present in the benzophenone-imines which has been subjected to extraction is adjusted by addition of the benzophenone-imines which is not subjected to extraction and then subjected to the oxidation of the step (a).

The benzophenone-imines that can be used as an extracting solvent in the above-described extraction may not necessarily be the same compound as benzophenone-imines to be subjected to the oxidation of the step (a) for obtaining benzophenone-azines, but they are desirably the same in industrial operation.

The copper halide catalyst thus recovered in an oily layer composed of the benzophenone-imines can be reused as a copper halide catalyst for the oxidation of benzophenone-imines with molecular oxygen with no need of any additional treatment.

Further, the extraction can be effected in either a batch system or a continuous system. In order to improve the extraction efficiency, counter-current multistage extraction can be appropriately adopted.

As described above, the present invention makes it possible to recover and reuse the copper halide catalyst by easy liquid-liquid extraction without requiring any extra treatment by conducting extraction and removal and back extraction and recovery in the absence of air; and to easily reuse the copper halide catalyst recovered even by the extraction in the presence of air by an additional operation of reduction. Thus, the present invention provides a process for producing benzophenone-azines with great industrial advantages.

The present invention will now be illustrated in greater detail with reference to examples. In these examples, Examples 1 to 13 show a process of recycling the catalyst according to the steps (a) to (c) of the present invention; Examples 14 to 49 and the Comparative Example show a treatment of the oxidation

reaction mixture obtained in the step (a) with a metal oxide catalyst; and Examples 50 to 87 show a treatment of the oxidation reaction mixture obtained in the step (a) with a carboxylic acid.

Example 1

(1) Production of Benzophenone-Azine by Oxidation of Benzophenone-Imine

To 500 g of benzophenone-imine (25.0% pure; the remainder was benzophenone)(imine: 0.691 mole) was added 1.95 g (19.7 mmol) of a cuprous chloride catalyst, and the resulting mixture was stirred at 140°C under atmospheric pressure 2 hours while blowing oxygen gas thereinto at a rate of 1.0 Nl/min. The resulting reaction mixture was found to contain benzophenone-imine and benzophenone-azine at concentrations of 3.0% and 21.5%, respectively.

(2) Extraction of Catalyst from Oxidation Reaction Mixture of Benzophenone-Imine

100 g of the reaction mixture obtained in (1) above and 50 g of each of the indicated aqueous solutions were brought into contact with each other at 90°C for 15 minutes in a nitrogen stream while stirring to extract the cuprous chloride catalyst. The results obtained are shown in Table 1.

TABLE 1

| Run No. | Type of Aqueous Solution | Amount of Cuprous Chloride in Aqueous Layer After Extraction |
|---|---|---|
| a | 2% HCl<br>10% $NH_4Cl$ | 3.8 mmol |
| b | 3% $H_2SO_4$<br>5% $NH_4Cl$<br>30% $(NH_4)_2SO_4$ | 3.6 mmol |

(3) Extraction of Catalyst with Benzophenone-Imine from Catalyst-Containing Aqueous Solution

To the whole amount of the cupruous chloride-containing aqueous solution obtained in (2)-a or b was added 10% aqueous ammonia or a 10% aqueous solution of hydrochloric acid to adjust to a pH of 4. The resulting aqueous solution and 25 g of a benzophenone-imine solution having the same composition as that used in (1) above were brought into contact with each other at 60°C for 3 minutes in a nitrogen stream while stirring to effect back extraction of the cuprous chloride catalyst. The results obtained are shown in Table 2.

TABLE 2

| Run No. | Type of Aqueous Solution | Amount of Cuprous Chloride Extracted in Organic Layer |
|---|---|---|
| A | aqueous solution obtained in (2)-a | 3.6 mmol |
| B | aqueous solution obtained in (2)-b | 3.4 mmol |

(4) Production of Benzophenone-Azine Using Extract of Catalyst with Benzophenone-Imine

An oxidation reaction was carried out in accordance with (1) above using each of the cuprous chloride catalysts obtained by back extractions (3)-A and B. That is, the oily solutions were adjusted so that 50 g of benzophenone-imine (25.0% pure; the remainder was benzophenone) (imine: 69.1 mmol) contains 0.195 g (1.97 mmol) of cuprous chloride catalyst, and oxygen gas was introduced therein at a rate of 0.1 N1/min at 140°C under atmospheric pressure for 2 hours while stirring. The results obtained are shown in Table 3.

TABLE 3

| Run No. | Recovery Method of Cuprous Chloride Catalyst | Composition of Reaction Mixture | |
|---|---|---|---|
| | | Imine | Azine |
| i | (3)-A | 3.1% | 21.4% |
| ii | (3)-B | 2.8% | 21.7% |

Examples 2 to 4

Extraction of Catalyst from Catalyst-Containing Aqueous Solution

50 g of an aqueous solution containing 4.0 mmol of a cuprous halide and 5.0 g of an ammonium halide was brought into contact with 25 g of a benzophenone-imine solution having the same composition as used in Example 1-(1) at 60°C in a nitrogen stream for 3 minutes while stirring. The results obtained are shown in Table 4.

TABLE 4

| Run No. | Type of Cuprous Halide | Type of Ammonium Halide | Amount of Cuprous Halide Extracted in Organic Layer |
|---|---|---|---|
| 2 | CuCl | $NH_4Cl$ | 3.8 mmol |
| 3 | CuBr | $NH_4Br$ | 3.5 mmol |
| 4 | CuI | $NH_4I$ | 3.6 mmol |

Examples 5 to 11

Extraction of Catalyst from Catalyst-Containing Aqueous Solution with Benzophenone-Imines

50 g of an aqueous solution containing 4.0 mmol of cuprous chloride and 5.0 g of ammonium chloride was brought into contact with 25 g of benzophenone-imines solution (25.0% pure; the remainder was the corresponding benzophenones) at 90°C in a nitrogen stream for 3 minutes while stirring. The results obtained are shown in Table 5.

TABLE 5

| Example No. | Type of Benzophenone-Imines | Cuprous Chloride Extracted in Organic Layer |
|---|---|---|
| 5 | 4-methylbenzophenone-imine | 3.8 mmol |
| 6 | 4-t-butylbenzophenone-imine | 3.6 mmol |
| 7 | 4-chlorobenzophenone-imine | 3.7 mmol |
| 8 | 4-phenylbenzophenone-imine | 3.7 mmol |
| 9 | 4-hydroxybenzophenone-imine | 3.0 mmol |
| 10 | 4-nitrobenzophenone-imine | 3.5 mmol |
| 11 | 4-methoxybenzophenone-imine | 3.4 mmol |

Example 12

Reduction of Cupric Chloride in Cupric Chloride-Containing Aqueous Solution to Cuprous Chloride and Extraction with Benzophenone-imine

5.3 g (1.06 mmol) of a 1% hydrazine hydrate aqueous solution was added to 50 g of an aqueous solution containing 4.0 mmol of cupric chloride and 5.0 g of ammonium chloride and having a pH of 3 at 90°C in a nitrogen stream, and the resulting mixture was stirred for 5 minutes. The mixture was adjusted to pH 6 with 5% aqueous ammonia under the same conditions and then brought into contact with 25 g of a benzophenone-imine solution having the same composition as used in Example 1-(1) at 60°C in a nitrogen stream for 3 minutes while stirring. It was found that 3.8 mmol of cuprous chloride was extracted in the benzophenone-imine layer.

Example 13

To 100 g of the oxidation reaction mixture obtained in Example 1-(1) was added 2.0 g of an activated clay powder, and the resulting mixture was stirred at 140°C under atmospheric pressure for 1 hour while blowing steam therein at a rate of 8.6 g/hr, thereby to decompose the unreacted benzophenone-imine in the oxidation reaction mixture into benzophenone and ammonia.

The activated clay powder was separated by filtration in a nitrogenous atmosphere. The filtrate contained benzophenone-imine at a concentration of 0.18%, and no change was observed in the benzophenone-azine concentration. Further, a part of the copper chloride catalyst was adsorbed onto the activated clay, and the filtrate contained 3.0 mmol of the copper catalyst.

The above filtrate was brought into contact with 50 g of an aqueous solution containing HCl and $NH_4Cl$ at concentrations of 2.0% and 10%, respectively, at 90°C for 15 minutes in a nitrogen stream while stirring to extract 2.9 mmol of the copper chloride catalyst in the aqueous layer.

To the resulting aqueous solution containing the copper chloride catalyst was added 10% aqueous ammonia to adjust the aqueous solution to a pH of 4. Thereafter, the aqueous solution was contacted with 20 g of a benzophenone-imine solution of the same composition as used in Example 1-(1) at 60°C for 3 minutes in a nitrogen stream while stirring. As a result, 2.7 mmol of cuprous chloride was extracted in the benzophenone-imine layer.

The cuprous chloride catalyst thus obtained by the above benzophenone-imine extraction was subjected to an oxidation reaction in accordance with Example 1-(1). That is, the benzophenone-imine solution was adjusted so that 50 g of benzophenone-imine (25.0% pure; the remainder was benzophenone) (imine: 69.1 mmol) contained 1.97 mmol of the cuprous chloride catalyst and then stirred at 140°C under atmospheric pressure for 2 hours while blowing oxygen gas therein at a rate of 0.1 nl/min. The oxidation reaction mixture was found to contain benzophenone-imine and benzophenone-azine at concentrations of 2.9% and 21.7%, respectively.

Example 14

100 g of a benzophenone-imine solution (23.5% pure; the remainder was benzophenone) and 0.39 g of commercially available cuprous chloride (manufactured by Koso Kagaku K.K.) were placed in a 200 ml-volume glass-made reaction vessel. Pure oxygen was introduced therein from a tube inserted to the bottom of the vessel under atmospheric pressure at a rate of 500 ml/min for 90 minutes while maintaining the reaction system at 140°C and stirring.

After 90 minutes, the reaction mixture was analyzed by gas chromatography to find that benzophenone-azine was produced in a yield of 87% and at a selectivity of 99% and that the content of the unreacted benzophenone-imine was 2.85%.

To 100 g of the above oxidation reaction mixture was then added 4.0 g of activated alumina in a powder form (manufactured by Nishio Kogyo K.K.) as an imine-hydrolyzing catalyst. Subsequently, water as superheated steam was blown thereinto from a ring sparger having three orifices of 0.5 mm in diameter inserted to the bottom of the reaction vessel at a rate of 8.6 g/hr for 1 hour while maintaining the reaction mixture at 140°C.

One hour later, the reaction mixture was analyzed by gas chromatography to find that the benzophenone-imine content decreased to 0.31%.

Further, the produced ammonia was withdrawn out of the vessel and absorbed in water. As a result of acid titration, ammonia in an amount corresponding to 96% of the decomposed amount of the imine was determined.

Examples 15 to 37

In the same manner as in Example 14, the oxidation of benzophenone-imine and hydrolysis of the unreacted benzophenone-imine in the resulting oxidation reaction mixture with water were carried out using various commercially available powdery oxides as catalysts. The content of the unreacted benzophenone-imine in the oxidation reaction mixture was 2.9% in each case. The results obtained are shown in Table 6.

TABLE 6

| Example No. | Catalyst | Amount of Catalyst (g) | % Hydrolysis of Imine (%) |
|---|---|---|---|
| 15 | boron trioxide ($B_2O_3$) | 4.0 | 48 |
| 16 | silicon oxide ($SiO_2$) | 2.0 | 90 |
| 17 | scandium oxide ($Sc_2O_3$) | 4.0 | 40 |
| 18 | titanium oxide ($TiO_2$) | 2.0 | 42 |
| 19 | vanadium pentoxide ($V_2O_5$) | 4.0 | 67 |
| 20 | gallium oxide ($Ga_2O_3$) | 4.0 | 38 |
| 21 | germanium oxide ($GeO_2$) | 4.0 | 35 |
| 22 | diarsenic pentoxide ($As_2O_5$) | 4.0 | 32 |
| 23 | yttrium oxide ($Y_2O_3$) | 4.0 | 50 |
| 24 | zirconium oxide ($ZrO_2$) | 4.0 | 59 |
| 25 | niobium oxide ($Nb_2O_5$) | 4.0 | 46 |
| 26 | indium oxide ($In_2O_3$) | 4.0 | 35 |
| 27 | stannic oxide ($SnO_2$) | 4.0 | 83 |
| 28 | antimony pentoxide ($Sb_2O_5$) | 4.0 | 86 |
| 29 | ferric oxide ($Fe_2O_3$) | 4.0 | 77 |
| 30 | tungsten oxide ($WO_2$) | 4.0 | 64 |
| 31 | bismuth oxide ($Bi_2O_3$) | 4.0 | 51 |
| 32 | cerium oxide ($CeO_2$) | 4.0 | 39 |
| 33 | thorium oxide ($ThO_2$) | 4.0 | 67 |
| 34 | activated clay | 2.0 | 94 |
| 35 | zeolite (for water treatment; manufactured by Kunimine Kogyo K.K.) | 2.0 | 49 |
| 36 | silica alumina ($Al_2O_3$: 13%) | 2.0 | 93 |
| 37 | silica zirconia ($ZrO_2$: 10%) | 2.0 | 84 |
| 38 | H-type Molecular Sieve 13X (conversion to $H^+$ type: 100%) | 2.0 | 90 |

Example 39

100 g of a benzophenone-imine solution (24.2% pure; the remainder was benzophenone) and 1.0 g of commercially available cuprous bromide (manufactured by Koso Kagaku K.K.) were placed in a 200 ml-volume glass-made reaction vessel, and pure oxygen was blown thereinto through a tube inserted to the bottom of the reaction vessel under atmospheric pressure at a rate of 500 ml/min for 100 minutes under stirring while maintaining the reaction mixture at 140°C.

100 minutes later, the reaction mixture was analyzed by gas chromatography to find that benzophenone-azine was produced in a yield of 87% at selectivity of 96%. The content of the unreacted benzophenone-imine was 2.3%.

To 100 g of the above-obtained oxidation reaction mixture was added 4.0 g of activated alumina in a powder form (manufactured by Nishio Kogyo K.K.) as an imine-hydrolyzing catalyst to effect hydrolysis of the benzophenone-imine under the same conditions as in Example 14.

The content of benzophenone-imine one hour later was found to be 0.35%.

Examples 40 to 46

In the same manner as in Example 39, oxidation of benzophenone-imine and hydrolysis of the unreacted benzophenone-imine in the resulting oxidation reaction mixture with water werre carried out using various commercially availabe powdery oxides. In each case, the oxidation reaction mixture contained 2.3% of the unreacted benzophenone-imine. The results obtained are shown in Table 7.

TABLE 7

| Example No. | Catalyst | | Hydrolysis of Imine (%) |
|---|---|---|---|
| | Type | Amount (g) | |
| 40 | vanadium pentoxide | 4.0 | 70 |
| 41 | zirconium oxide | 4.0 | 62 |
| 42 | tungsten oxide | 4.0 | 65 |
| 43 | silica alumina | 4.0 | 91 |
| 44 | silica magnesia | 4.0 | 82 |
| 45 | acid clay | 2.0 | 74 |
| 46 | activated clay | 2.0 | 93 |

Example 47

50 g of a benzophenone-imine solution (46.7% pure; the remainder was benzophenone), 50 g of o-dichlorobenzene (manufactured by Koso Kagaku K.K.) and 1.0 g of cuprous chloride were charged in a reaction vessel, and oxygen gas was introduced therein from the bottom of the vessel at a rate of 500 ml/min at 120°C for 60 minutes while stirring.

Gas chromatography of the reaction mixture revealed that benzophenone-azine was produced in a yield of 87% at a selectivity of 99% and that the unreacted imine content was 2.83%.

To the thus-obtained oxidation reaction mixture was added 4 g of powdery activated clay (manufactured by Mizusawa Kagaku K.K.) as an imine-hydrolyzing catalyst, and then water as superheated steam was introduced therein at a rate of 7.2 g/hr for 1 hour while stirring.

The benzophenone-imine content after the reaction was decreased to 0.09%.

Example 48

In the same manner as in Example 14 but using 100 g of 4-methylbenzophenone-imine (28.4% pure; the remainder was methylbenzophenone), oxidation and hydrolysis of the unreacted imine was carried out.

The imine content after the oxidation was 2.5% and that after the imine hydrolysis was 0.27%.

Example 49

In the same manner as in Example 14 but using 100 g of a 4-*t*-butylbenzophenone-imine solution (21.5% pure; the remainder was 4-t-butylbenzophenone), oxidation and hydrolysis of the unreacted imine were carried out.

The imine contents after the oxidation and the hydrolysis of the imine were 2.0% and 0.25%, respectively.

Comparative Example

Benzophenone-imine was oxidized under the same conditions as in Example 14 to obtain a reaction mixture containing benzophenone-azine produced in a yield of 86.5% and 3.05% of the unreacted benzophenone-imine.

Hydrolysis of the imine was attempted under the same conditions as in Example 14 but without adding any imine-hydrolyzing catalyst. After one hour's reacting, the imine content of the reaction mixture was found to be 3.01%, which indicated that the imine was not substantially hydrolyzed.

Example 50

100 g of a benzophenone-imine solution (23.5% pure; the remainder was benzophenone) and 0.40 g of commercially available cuprous chloride (manufactured by Koso Kagaku K.K.) were charged in a reaction vessel, and pure oxygen was introduced into the mixture through a tube inserted to the bottom of the vessel under atmospheric pressure at a rate of 400 ml/min at 140°C for 90 minutes under stirring.

The reaction mixture 90 minutes later was analyzed by gas chromatography to find that benzophenone-azine was produced in a yield of 87% at a selectivity of 99% and that the unreacted benzophenone-imine content was 2.85%.

After completion of the oxidation reaction, 122 mg of benzoic acid of reagent grade (manufactured by Koso Kagaku K.K.) was added to the reaction mixture as an imine-hydrolyzing catalyst. While maintaining the mixture at 140°C under atmospheric pressure, water was introduced therein as superheated steam through a tube inserted to the bottom of the reaction vessel at a rate of 4.5 g/hr for 1 hour under stirring. One hour later, the reaction mixture was analyzed by gas chromatography to find that the benzophenone-imine content was decreased to 0.23%. The ammonia produced was withdrawn out of the reaction vessel, absorbed in water, and titrated with an acid. As a result, ammonia in am amount corresponding to 92% of the hydrolyzed imine was determined.

Examples 51 to 83

In the same manner as in Example 49, an oxidation reaction was carried out, and subsequently, the unreacted benzophenone-imine was hydrolyzed using various carboxylic acids as catalysts. The results obtained are shown in Table 8.

TABLE 8

| Example No. | Carboxylic Acid Type | Carboxylic Acid Amount | Hydrolysis of Imine |
|---|---|---|---|
| | | (mg) | (%) |
| 51 | acetic acid | 60 | 77 |
| 52 | propionic acid | 72 | 42 |
| 53 | butyric acid | 88 | 56 |
| 54 | isobutyric acid | 88 | 40 |
| 55 | valeric acid | 102 | 64 |
| 56 | pivalic acid | 102 | 59 |
| 57 | enanthic acid | 130 | 83 |
| 58 | caprylic acid | 144 | 87 |
| 59 | palmitic acid | 157 | 88 |
| 60 | stearic acid | 185 | 94 |
| 61 | monochloroacetic acid | 95 | 86 |
| 62 | trifluoroacetic acid | 114 | 63 |
| 63 | lactic acid | 90 | 35 |
| 64 | oxalic acid | 90 | 44 |
| 65 | citric acid | 210 | 38 |
| 66 | cyclopentanecarboxylic acid | 114 | 89 |

TABLE 8 (continued)

| Example No. | Carboxylic Acid Type | Amount (mg) | Hydrolysis of Imine (%) |
|---|---|---|---|
| 67 | salicylic acid | 138 | 89 |
| 68 | alanine | 89 | 41 |
| 69 | methionine | 149 | 60 |
| 70 | ethylenediamine-tetraacetic acid | 296 | 85 |
| 71 | trimellitic acid | 210 | 79 |
| 72 | *p*-toluylic acid | 136 | 90 |
| 73 | nicotinic acid | 123 | 93 |
| 74 | isophthalic acid | 166 | 89 |
| 75 | lutidinic acid | 167 | 92 |
| 76 | trimesic acid | 210 | 73 |
| 77 | caproic acid | 173 | 85 |
| 78 | α-naphthylacetic acid | 187 | 92 |
| 79 | azelaic acid | 173 | 91 |
| 80 | ammonium benzoate | 139 | 92 |
| 81 | benzoyl chloride | 141 | 87 |
| 82 | dimethyl isophthalate | 199 | 51 |
| 83 | phthalimide | 147 | 68 |

Examples 84 to 86

Oxidation and hydrolysis of benzophenone-imine were carried out in the same manner as in Example 49 except for changing the type of the starting benzophenone. The results obtained are as shown in Table 9.

TABLE 9

| Example No. | Benzophenone | Unreacted Imine in Oxidation Reaction Mixture | Hydrolysis of Imine |
|---|---|---|---|
| 84 | 4-methylbenzophenone | 3.56 | 93 |
| 85 | 4-t-butylbenzophenone | 3.14 | 90 |
| 86 | 4-chlorobenzophenone | 2.52 | 87 |

Example 87

30 g of benzophenone-imine (46.7% pure; the remainder was benzophenone), 30 g of *o*-dichlorobenzene (manufactured by Koso Kagaku K.K.) and 0.50 g of cuprous chloride were placed in a reaction vessel, and oxygen gas was blown to the mixture from the bottom of the reaction vessel at a rate of 300 ml/min for 60 minutes under stirring while maintaining the mixture at 120°C.

Gas chromatography of the reaction mixture revealed that benzophenone-azine was produced in a yield of 85% at a selectivity of 99% and that the unreacted benzophenone-imine content was 3.3%.

To the resulting oxidation mixture was added 61 mg of benzoic acid, and water in the state of steam was then introduced therein from the bottom under atmospheric pressure at a rate of 6.0 g/hr at 120°C for 1 hour while stirring.

The benzophenone-imine content after the reaction was 0.16%.

**Claims**

1. A process of producing a benzophenone-azine wherein:

(a) a benzophenone-imine is oxidized with molecular oxygen in the presence of at least one copper halide catalyst to produce a benzophenone-azine, and

(b) the oxidation reaction mixture obtained in the step (a) is brought into contact with an inorganic aqueous solution to extract and remove the catalyst;

characterized in that in step (b) the aqueous extractant solution contains (i) a mineral acid at a concentration of 0.1 to 10% by weight and in an amount of 0.5 to 5.0 equivalents based on the benzophenone-imine fed and (ii) an inorganic halide at a concentration of 0.1 to 50% by weight and that

(c) the catalyst-containing aqueous solution obtained in the step (b) is brought into contact with benzophenone-imine, which may at least partly obtained from that fed to step (a), to extract the catalyst, and the catalyst-containing benzophenone-imine solution obtained is recycled to the step (a) in the form of a benzophenone-imine solution.

2. A process as claimed in Claim 1, wherein the mineral acid used in step (b) is hydrochloric acid, hydrobromic acid, hydroiodic acid, sulfuric acid, phosphoric acid, carbone acid or nitric acid.

3. A process as claimed in Claim 1 or 2, wherein the inorganic halide used in step (b) is ammonium chloride, ammonium bromide, ammonium iodide or a chloride, bromide or iodide of lithium, sodium, potassium, beryllium, magnesium, calcium or aluminium.

4. A process as claimed in any of Claims 1 to 3, wherein the extraction in step (b) is carried out under a pressure of 0.1 to 10 atmospheres.

5. A process as claimed in any of Claims 1 to 3, wherein the extraction in step (b) is carried out in the absence of air.

6. A process as claimed in any preceding claim, wherein the oxidation reaction mixture obtained in step (a) contains the unreacted benzophenone-imine, and the process further comprises hydrolyzing said unreacted benzophenone-imine into a benzophenone and ammonia in the presence of an imine-hydrolyzing catalyst and treating the resulting reaction mixture in step (b).

7. A process as claimed in Claim 6, wherein the unreacted benzophenone-imine is hydrolyzed by feeding water or steam into the oxidation reaction mixture obtained in the step (a) and heating the resulting mixture at a temperature of 60°C to 350°C in the presence of at least one metal oxide or a carboxylic acid.

8. A process as claimed in any preceding claim, wherein the catlayst-containing aqueous solution in step (c) contains a salt of a divalent copper and the process further includes reducing a part or the whole of said cupric salt to a cuprous salt, which is then contacted with a benzophenone-imine.

9. A process as claimed in any preceding claim, wherein the extraction in step (c) is carried out in the absence of air.

10. A process as claimed in any preceding claim, wherein the copper halide catalyst in step (a) is present at a concentration of $10^{-4}$ to 2 mols per litre of the total volume of the reaction mixture.

11. A process as claimed in any preceding claim, wherein the oxidation in the step (a) is carried out while controlling the conversion of benzophenone-imines so that the molar ratio of benzophenone-imine to copper halide is maintained at 1.5 or more.

**Patentansprüche**

1. Verfahren zur Herstellung eines Benzophenon-azins, bei dem

(a) ein Benzophenon-imin mit molekularem Sauerstoff in Gegenwart von wenigstens einem Kupferhalogenid-Katalysator unter Bildung eines Benzophenon-azins oxidiert wird und

(b) das in der Stufe (a) erhaltene Oxidations-Reaktions-Gemisch in Kontakt mit einer anorganischen, wässrigen Lösung zum Extrahieren und Entfernen des Katalysators gebracht wird,

dadurch gekennzeichnet, dass in Stufe (b) die wässrige Extrahierlösung (i) eine Mineralsäure in einer Konzentration von 0,1 bis 10 Gew.% und in einer Menge von 0,5 bis 5,0 Äquivalenten, bezogen auf das zugeführte Benzophenon-imin, und (ii) ein anorganischen Halogenid in einer Konzentration von 0,1 bis 50 Gew.% enthält, und dass

(c) die in Stufe (b) erhaltene katalysatorhaltige, wässrige Lösung in Kontakt mit Benzophenon-imin gebracht wird, welches wenigstens zum Teil von dem in der Stufe (a) zugeführten erhalten wurde, um den Katalysator zu extrahieren und dass die erhaltene katalysatorhaltige Benzophenon-imin-Lösung in die Stufe (a) in Form einer Benzophenon-imin-Lösung zurückgeführt wird.

2. Verfahren gemäss Anspruch 1, bei dem die in Stufe (b) verwendete Mineralsäure Chlorwasserstoffsäure, Bromwasserstoffsäure, Jodwasserstoffsäure, Schwefelsäure, Phosphorsäure, Kohlensäure oder Salpetersäure ist.

3. Verfahren gemäss Ansprüchen 1 oder 2, bei dem das in Stufe (b) verwendete anorganische Halogenid Ammoniumchlorid, Ammoniumbromid, Ammoniumjodid oder ein Chlorid, Bromid oder Jodid von Lithium, Natrium, Kalium, Beryllium, Magnesium, Calcium oder Aluminium ist.

4. Verfahren gemäss einem der Ansprüche 1 bis 3, bei dem die Extraktion in Stufe (b) unter einem Druck von 0,1 bis 10 Atm durchgeführt wird.

5. Verfahren gemäss einem der Ansprüche 1 bis 3, bei dem die Extraktionsstufe in (b) in Abwesenheit von Luft durchgeführt wird.

6. Verfahren gemäss einem der vorhergehenden Ansprüche, bei dem das in Stufe (a) erhaltene Oxidations-Reaktionsgemisch das nicht-umgesetzte Benzophenon-imin enthält und das Verfahren weiterhin das Hydrolysieren des nicht-umgesetzten Benzophenon-imins zu einem Benzophenon und Ammoniak in Gegenwart eines Imin-Hydrolysierungskatalysators und das Behandeln der erhaltenen Reaktionsmischung in Stufe (b) umfasst.

7. Verfahren gemäss Anspruch 6, bei dem das nicht-umgesetzte Benzophenon-imin hydrolysiert wird, indem man Wasser oder Wasserdampf in das in Stufe (a) erhaltene Oxidations-Reaktionsgemisch einführt und die erhaltene Mischung bei einer Temperatur von 60 bis 350°C in Gegenwart wenigstens eines Metalloxids oder einer Carbonsäure erhitzt.

8. Verfahren gemäss einer der vorhergehenden Ansprüche, bei dem die katalysatorhaltige wässrige Lösung in Stufe (c) ein Salz des zweiwertigen Kupfers enthält und das Verfahren weiterhin das Reduzieren eines Teils oder der Gesamtmenge des Kupfer (II) salzes zu einem Kupfer(I)salz, welches dann mit einem Benzophenon-imin in Kontakt gebracht wird, einschliesst.

9. Verfahren gemäss einem der vorhergehenden Ansprüche, bei dem die Extraktion in Stufe (c) in Abwesenheit von Luft durchgeführt wird.

10. Verfahren gemäss einem der vorhergehenden Ansprüche, bei dem der Kupferhalogenid-Katalysator in Stufe (a) in einer Konzentration von $10^{-4}$ bis 2 Mol pro Liter des Gesamtvolumens des Reaktionsgemisches vorliegt.

11. Verfahren gemäss einem der vorhergehenden Ansprüche, bei dem die Oxidation in der Stufe (a) unter Überwachung der Umwandlung des Benzophenon-imins durchgeführt wird, so dass das Molverhältnis von Benzophenon-imin zu Kupferhalogenid bei 1,5 oder mehr gehalten wird.

**Revendications**

1. Procédé pour la production d'une benzophénone-azine dans lequel;

(a) on oxyde une benzophénone-imine par l'oxygène moléculaire en présence d'au moins un catalyseur d'halogénure de cuivre pour produire une benzophénone-azine et

(b) on met en contact le mélange d'oxydation obtenu sur l'étape (a) avec une solution aqueuse inorganique pour extraire et séparer le catalyseur, caractérisé en ce que, dans l'étape (b), la solution aqueuse d'extraction contient: (i) un acide minéral à une concentration de 0,1 à 10% en poids et en quantité de 0,5 à 5,0 d'équivalents par rapport à la benzophénone-imine introduite; et (ii) un halogénure inorganique à une concentration de 0,1 à 50% en poids; et en ce que

(c) on met en contact la solution aqueuse contenant le catalyseur obtenue dans l'étape (b) avec la benzophénone-imine qui peut être obtenue au moins partiellement à partir de celle introduite dans l'étape (a), pour extraire le catalyseur et on recycle la solution de benzophénone-imine contenant le catalyseur obtenu à l'étape (a) sous la forme d'une solution de benzophénone-imine.

2. Procédé selon la revendication 1, dans lequel l'acide minéral utilisé dans l'étape (b) est l'acide chlorhydrique, l'acide bromhydrique, l'acide iohydrique, l'acide sulfurique, l'acide phosphorique, l'acide carbonique ou l'acide nitrique.

3. Procédé selon la revendication 1 ou 2, dans lequel l'halogénure inorganique utilisé dans l'étape (b) est le chlorure d'ammonium, le bromure d'ammonium, l'iodure d'ammonium ou un chlorure, bromure ou iodure de lithium, sodium, potassium, béryllium, magnésium, calcium ou aluminium.

4. Procédé selon l'une quélconque des revendications 1 à 3, dans lequel l'extraction dans l'étape (b) est mise en oeuvre sous une pression de 0,1 à 10 atmosphères.

5. Procédé selon l'une quelconque des revendications 1 à 3, dans lequel l'extraction dans l'étape (b) est mise en ouevre en l'absence d'air.

6. Procédé selon l'une quelconque des revendications précédentes, dans lequel le mélange de réaction d'oxydation obtenu dans l'étape (a) contient la benzophénone-imine n'ayant pas réagi et le procédé comprend en outre l'hydrolyse de ladite benzophénone-imine n'ayant pas réagi en benzophénone et ammoniac en présence d'un catalyseur d'hydrolyse de l'imine et le traitement du mélange de réaction résultant dans l'étape (b).

7. Procédé selon la revendication 6, dans lequel la benzophénone-imine n'ayant pas réagi est hydrolysée par introduction d'eau ou de vapeur dans le mélange de réaction d'oxydation obtenu dans l'étape (a) et chauffage du mélange résultant à une température de 60 à 350°C en présence d'au moins un oxyde métallique ou un acide carboxylique.

8. Procédé selon l'une quelconque des revendications précédentes, dans lequel la solution aqueuse contenant le catalyseur dans l'étape (c) contient un sel de cuivre divalent et le procédé comprend en outre la réduction d'une partie ou de la totalité dudit sel cuivrique en un sel cuivreux, qui est ensuite mis en contact avec une benzophénone-imine.

9. Procédé selon l'une quelconque des revendications précédentes, dans lequel l'extraction dans l'étape (c) est mise en oeuvre en l'absence d'air.

10. Procédé selon l'une quelconque des revendications précédentes, dans lequel le catalyseur d'halogénure de cuivre dans l'étape (a) est présent à une concentration de $10^{-4}$ à 2 mol/l du volume total du mélange de réaction.

11. Procédé selon l'une quelconque des revendications précédentes, dans lequel l'oxydation dans l'étape (a) est mise en oeuvre en contrôlant la conversion des benzophénone-imines de telle sorte que le rapport molaire de la benzophénone-imine à l'halogénure de cuivre soit maintenu à 1,5 ou plus.